# EUROPEAN PATENT APPLICATION

(11) **EP 4 653 529 A1**
(43) Date of publication of application: **26.11.2025**
(21) Application number: 24744918.4
(22) Date of filing: 19.01.2024
(51) Int. Cl.: C12N 15/11, C12N 15/85

(54) **RNA HAVING LARIAT-CAP STRUCTURE FOR IMPROVING INTRACELLULAR STABILITY AND BIOGENESIS OF MRNA, AND USE THEREOF**

(30) Priority: 20.01.2023 KR 20230008494
(71) Applicant: Korea Advanced Institute of Science and Technology, Daejeon 34141 (KR)
(72) Inventor: KIM, Yoon Ki, Daejeon 34141 (KR); CHANG, Jeeyoon, Daejeon 34141 (KR)
(74) Representative: Schnappauf, Georg
(86) International application number: PCT/KR2024/000938
(87) International publication number: WO 2024/155139

(57) **Abstract**

Unlike DNA, mRNA has the advantage of enabling transient expression of a desired protein directly in the cytoplasm without having to enter the nucleus, and thus attempts to use mRNA vaccines are actively underway. However, mRNA has lower structural stability than DNA, and thus has limitations with respect to industrial application. Therefore, the present invention relates to a technology for remarkably improving intracellular stability and biogenesis of mRNA. An mRNA stabilization method and an mRNA stabilization composition, of the present invention, have the excellent effects of enhancing target nucleic acid stabilization and target protein expression in mRNA vaccines and the like, and thus are expected to be widely used in the health/medical field.

## Description

### [Technical Field]

The present disclosure relates to a sequence for stabilizing nucleic acids, specifically an RNA sequence of a lariat cap structure that enhances the intracellular stability and biosynthesis of mRNA, and a composition for stabilizing nucleic acids including the same.

### [Background Art]

Messenger RNA (mRNA) has the advantage that desired proteins can be expressed temporarily directly in the cytoplasm without the need to enter the nucleus, unlike DNA, so it is attracting attention as a new treatment method in the health/medical field. In particular, global biotech and pharmaceutical companies are actively trying to use mRNA vaccines for disease treatment and prevention, for example, in the case of new infectious diseases, changing only the nucleotide sequence of mRNA makes it easy to produce vaccines quickly, and mRNA used as a vaccine is removed from human cells after expressing proteins within a short time, so it can be expected to be safer than DNA vaccines. However, mRNA has a lower structural stability than DNA, so it has limitations in being used industrially. In the case of mRNA vaccines, which were first attempted in COVID-19, the half-life is unstable at about 4 hours, so about 30~100ug of mRNA is needed to be administered at one time for sufficient protein expression, and this single administration of a large amount of mRNA has become a cause of various side effects depending on the individual.

Therefore, the present invention has been devised for the solution of the above problems, and relates to a technology that significantly improves the intracellular stability and biosynthesis of mRNA. The mRNA stabilization method and mRNA stabilization composition of the present invention have excellent effects of target nucleic acid stabilization and enhanced expression of target proteins in mRNA vaccines, etc., so it is expected to be widely used in the health/medical field.

### [Disclosure]

### [Technical Problem]

One object of the present disclosure is to provide a method for stabilizing a desired nucleic acid, particularly mRNA.

Another object of the present disclosure is to provide a vector for improving the stability of a desired nucleic acid, particularly mRNA.

Still another object of the present disclosure is to provide a composition for stabilizing a desired nucleic acid, particularly mRNA.

However, objects to be achieved by the present disclosure are not limited to the objects mentioned above, and other objects not mentioned above may be clearly understood by those skilled in the art from the following description.

### [Technical Solution]

Hereinafter, various embodiments described herein will be described with reference to figures. In the following description, numerous specific details are set forth, such as specific configurations, compositions, and processes, etc., in order to provide a thorough understanding of the present disclosure. However, certain embodiments may be practiced without one or more of these specific details, or in combination with other known methods and configurations. In other instances, known processes and preparation techniques have not been described in particular detail in order to not unnecessarily obscure the present disclosure. Reference throughout this specification to "one embodiment" or "an embodiment" means that a particular feature, configuration, composition, or characteristic described in connection with the embodiment is included in at least one embodiment of the present disclosure. Thus, the appearances of the phrase "in one embodiment" or "an embodiment" in various places throughout this specification are not necessarily referring to the same embodiment of the present disclosure. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present disclosure pertains.

In the present invention, the term "lariat structure" refers to a type of structure found in the process of splicing of the precursor of mRNA in the nucleus, in which a lariat-type intron is always produced as a byproduct of splicing, and the produced lariat intron is mostly removed from the nucleus. As a related concept, the term "lariat cap" in the present invention means that an RNA in a form similar to a lariat structure artificially produced as a result of splicing is formed at the 5' end of a target sequence to be expressed through an in vitro transcription process. The RNA sequence GIR1, which exists in the myxomycete *Didymium iridis* species, is known to perform the function of a ribozyme, and in particular, this RNA sequence induces a very unusual reaction that directly connects the first and third nucleotide sequences of RNA. As a result, a linear RNA with a lariat-cap structure in which the first and third sequences of the 5' end are connected can be produced, rather than a complete linear RNA form. The lariat cap in the present invention means that a lariat structure cap is formed at the 5' end of a target sequence to be expressed using the above-described lariat-cap forming sequence. However, as a means of doing so, the ribozyme is not limited to GIR1 or a gene sequence represented by any one of SEQ ID NOs: 1 to 3 used in the present invention, and any sequence capable of forming a lariat structure cap can be used without limitation.

In this invention, the term "nucleic acid" includes the concepts of DNA and RNA. It is used in the same sense as the term "polynucleotide". Preferably, it is a polymer made up of nucleotide monomers that are covalently bonded to each other by phosphodiester bonds in the sugar/phosphate backbone. Alternatively, it may include base-modified, sugar-modified, or backbone-modified DNA or RNA. However, considering the purpose of this invention, it is preferable that the nucleic acid in this invention refers to RNA, particularly mRNA. As a related concept, the nucleic acid intended to be expressed in this invention may mean the same as the target sequence intended to be expressed, and may be the target mRNA sequence that we want to enhance stability with the nucleic acid stabilization method or nucleic acid stabilization composition of this invention.

In this invention, the term "stabilizing nucleic acid" refers to the concept of preventing the collapse or deformation of the nucleic acid structure in a cell, or outside a cell, to stabilize the nucleic acid structure. In general, RNA is made up of a single strand (single strand) and has a higher degree of activity compared to DNA, so it has a lower structural stability. Therefore, the nucleic acid stabilization in the present invention is intended to prevent structural collapse or deformation of DNA, or RNA, preferably RNA, and more preferably mRNA, and the method or composition using a sequence capable of forming a lariat structure cap according to the present invention to delay the structural collapse or deformation of the expressed nucleic acid.

In this invention, the term "sequence homology" is a percentage indicating the degree to which two compared sequences are identical. The sequences to be compared to determine the degree of homology are preferably of the same length, but if the sequence length differs, the sequence with the longer length is used as the basis for calculation. For example, a 10 nt sequence has 80% homology with an 8 nt sequence that is expressed as an identical sequence as a part of it. In the present invention, the ribozyme used as a means for the nucleic acid stabilization effect of the present invention may be a sequence selected from any one of SEQ ID NOs: 1 to 3, or a sequence having 70% or more homology with any one of SEQ ID NOs: 1 to 3. Alternatively, it may be a sequence having 80% or more homology with any one of SEQ ID NOs: 1 to 3. Alternatively, it may be a sequence having 90% or more homology with any one of SEQ ID NOs: 1 to 3. Alternatively, it may be a sequence having 95% or more homology with any one of SEQ ID NOs: 1 to 3. The homology is not limited to a specific percentage, and as long as the characteristic of forming a lariat-cap is preserved, any sequence can be used without limitation.

In this invention, the term "artificial nucleic acid" may be understood as a non-natural nucleic acid molecule that does not exist in nature. The artificial nucleic acid may be composed of 100% of a sequence that does not exist in nature, or may be composed of a mixture of a part of a sequence that exists in nature (wild type) and a part of a sequence that does not exist in nature. In the case where it is composed only of a sequence that exists in nature, the sequence may be a mixture of sequences derived from different species. In the case where it is composed of a mixture of a part of a sequence that exists in nature and a part of a sequence that does not exist in nature, the sequence that exists in nature may be a mixture of sequences derived from one or more species. The artificial nucleic acid may be non-natural because of a modification of its individual sequence that does not occur naturally, for example, a structural modification of a nucleotide that does not occur naturally. In addition, the artificial nucleic acid may be a DNA molecule, an RNA molecule, or a hybrid molecule containing DNA and RNA parts. In the present invention, the nucleic acid used for stabilizing mRNA, in particular, may be one in which artificial modifications are made to a sequence derived from a single species, or an artificial nucleic acid in which sequences derived from different species are mixed. Specifically, it may be one in which a natural ribozyme sequence derived from one species is mixed with a sequence coding for an internal ribosome entry site (IRES) derived from another species. In this case, the sequence for the formation of the lariat-cap may be any one or more of sequence represented by any one of SEQ ID NOs: 1 to 3, and the IRES may be a sequence known in the art, or may be represented by SEQ ID NOs: 4. The artificial nucleic acid can be understood as a type of vector. In the context of the present invention, the vector may include an artificial nucleic acid selected for nucleic acid stabilization, specifically, an artificial nucleic acid in which a sequence coding for an internal ribosome entry site (IRES) is mixed with a natural ribozyme sequence derived from a species. In such a case, the natural ribozyme sequence derived from a species and the sequence coding for the internal ribosome entry site derived from another species within a single vector may be arranged in connection with each other, or arranged at a certain interval. Such a vector may be a storage vector, an expression vector, a cloning vector, or a transport vector. A storage vector is a vector that allows for the convenient storage of a nucleic acid molecule, such as an mRNA molecule. An expression vector may be used for the production of an expression product, such as RNA, for example, mRNA, or a peptide, polypeptide or protein. A cloning vector is a vector that typically includes a cloning site that can be used to merge a nucleic acid sequence into the vector. A cloning vector may be, for example, a plasmid vector or a bacteriophage vector. A transport vector is a vector suitable for transporting a nucleic acid molecule into a cell or organism, and may be, for example, a viral vector. In the context of the present invention, the vector may be, for example, an RNA vector or a DNA vector. Preferably, the vector of the present invention is a plasmid vector, or a viral vector, but is not limited thereto. The vector described above can be used to introduce the desired DNA or RNA (for example, mRNA) nucleic acid into a cell, preferably a eukaryotic cell, to change the original characteristics of the introduction object. This process is called transfection. Transfection can be performed using any method known to a person skilled in the art for introducing a nucleic acid molecule into a cell, preferably a eukaryotic cell such as a mammalian cell. These methods include, for example, electroporation, lipofection based on cationic lipids and/or liposomes, calcium phosphate precipitation, nanoparticle-based transfection, virus-based transfection, or cationic polymer-based transfection such as DEAE-dextran or polyethyleneimine. Alternatively, transfection can be performed using a virus, such as a lentivirus. The introduction object whose original characteristics have been changed by transfection is called a transfectant. The transfectant may be a virus, bacteria, plant cell, or animal cell.

The artificial nucleic acid, or nucleic acid stabilization composition of the present invention may be used for immunity-boosting of a desired object. The immunity-boosting refers to an amplified immune response desired for a selected antigen such as a characteristic component of a bacterial surface, a viral particle, a tumor antigen, etc. It is possible to induce an immune response to be amplified naturally or artificially, and in the case of artificial immune response amplification, vaccination is the most common method. A vaccine is typically understood as a preventive or therapeutic substance that provides at least one antigen, preferably an immunogen. The antigen or immunogen may be derived from any substance suitable for vaccination. For example, the antigen or immunogen may be derived from a bacterium or viral particle, or from a tumor or cancer tissue. The antigen or immunogen stimulates the adaptive immune system in vivo. Conventionally, vaccines have been dominated by the use of proteins or peptides derived from antigens or immunogens as stimulants for the adaptive immune system in vivo, but recent attempts have been made to use nucleic acids as stimulants. In particular, mRNA has the advantage of being able to be expressed as a desired protein immediately in the cytoplasm without the need to enter the nucleus, unlike DNA, so it is attracting attention as a new treatment method in the health/medical field. For example, for emerging infectious diseases, by changing only the nucleotide sequence of mRNA, it is easy to manufacture vaccines quickly, and mRNA used as a vaccine can express proteins inside human cells and then removed in a short time, so it can be expected to be safe compared to DNA vaccines. However, mRNA has a lower stability than DNA, making it difficult to use industrially. In the case of mRNA vaccines first attempted in COVID-19, the half-life is about 4 hours, making it unstable and requiring about 30-100ug of mRNA for a single dose to express enough protein, which can cause various side effects depending on the individual. Therefore, from the perspective of solving these conventional problems, an artificial nucleic acid that includes a sequence for forming a lariat-cap having excellent nucleic acid stabilization effect, or a sequence coding for internal ribosome entry site (IRES) in addition to a sequence for forming a lariat-cap can be administered as a vaccine with a nucleic acid derived from a suitable antigen or immunogen for immune enhancement. In this case, a sequence for forming a lariat-cap is preferably located at the 5' side of the antigen genetic information in the nucleic acid vaccine, and a sequence coding for internal ribosome entry site (IRES) is preferably located between a sequence for forming a lariat-cap and antigen genetic information. Furthermore, it improves the stability of nucleic acid vaccines and allows antigens to be efficiently synthesized in vivo, so even with little vaccine administration, high effects can be expected for disease treatment and prevention. In addition, as the dose of the vaccine being administered decreases to meet the expected treatment effect, it is possible to reduce the production cost of the vaccine. The vaccine described may additionally include an adjuvant (component) for boosting the expected effect. An adjuvant is typically a pharmaceutical and/or immunological agent that can modify or enhance the effect of another agent, such as a drug or vaccine. This is to be interpreted broadly and covers a wide range of substances. Typically, these substances can increase the immunogenicity of an antigen. For example, an adjuvant can be recognized by the innate immune system and induce an innate immune response. The use of the artificial sequence of the present invention, which includes a sequence for lariat-cap formation together with a nucleic acid intended for expression as an antigen, or the vaccine, can be infinitely extended depending on the type of antigen or immunogen included. For example, if the nucleic acid is derived from a virus, the vaccine is a vaccine for that virus. For example, if the antigen or immunogen is derived from the SARS-CoV-2 virus, the vaccine is a vaccine for coronavirus disease (COVID-19). If the antigen or immunogen is derived from the influenza virus (A, B, or C), the vaccine is a flu vaccine. If the antigen or immunogen is derived from the papillomavirus or papillomavirus, the vaccine is a cervical cancer vaccine.

The artificial nucleic acid or nucleic acid stabilizing composition of the present invention may be used as a pharmaceutical composition for preventing or treating a target disease by enhancing the immunity of a target entity.

The artificial nucleic acid or nucleic acid stabilizing composition of the present invention may be used as a pharmaceutical composition for preventing or treating a target disease by enhancing the immunity of a target entity. Herein, a genetic disorder refers to the same meaning as genetic disease caused by genetic defect and refers to a disease encompassing abnormalities in DNA base sequences, which are the substrates of genes. It may be divided into autosomal gene defect diseases and sex chromosome gene defect diseases, and each may be divided into recessive trait defect diseases and dominant trait defect diseases. Without being limited thereto, as specific examples, autosomal recessive trait defect diseases include phenylketonuria(PKU), sickle cell anemia, cystic fibrosis, albinism, xeroderma pigmentosum, Wilson's disease, Hurler-Scheie syndrome, Tay-Sachs disease, typical hemochromatosis (type 1), and Gitelman syndrome, and autosomal dominant trait defect diseases include neurofibromatosis type 1 (NF-1), autosomal dominant polycystic kidney disease (ADPKD), Huntington's disease, muscular dystrophy, fatal familial insomnia (FFI), Charcot Marie Tooth disease (CMT), and Treacher collins syndrome, and sex chromosome recessive trait defect diseases include Duchenne muscular dystrophy (DMD), hemophilia, X-linked Agammaglobulinema (XLA), testicular feminization syndrome, color blindness, azoospermia, and primary immune deficiency syndrome including selective IgA deficiency, panhypogammaglobulinemia, DiGeorge syndrome, severe combined immunodeficiency, hyper-IgE syndrome, hyper-IgM syndrome, Wiskott-Aldrich syndrome, chronic granulomatous disease, or congenital neutropenia. Sex chromosome dominant trait defect diseases include congenital systemic hirsutism and hereditary hair loss. The pharmaceutical composition of the present invention may be used for the treatment of the genetic disorder caused by genetic defect.

Furthermore, the pharmaceutical composition herein refers to a composition administered for the prevention or treatment of a desired disease. The disease may be any disease that requires prevention or treatment, but for the purpose of the present invention, it may be a disease that is prevented or treated by regulation of gene expression. The gene herein may be a gene that directly affects the prevention or treatment of a disease, such as a genetic disease, or a gene that indirectly affects the prevention or treatment of a disease through, for example, immune cell stimulation. The pharmaceutical composition may be in the form of a capsule, tablet, granule, injection, ointment, powder, or beverage, and may be intended for humans. The pharmaceutical composition of the present invention may be formulated into various dosage forms such as powders, granules, capsules, tablets, aqueous suspensions, etc. for oral administration, topical administration, suppositories, and injections, but is preferably a suppository or injection. In addition, the pharmaceutical composition of the present invention may contain a pharmaceutically acceptable carrier. Pharmaceutically acceptable carriers for oral administration may include binders, lubricants, disintegrants, diluents, solubilizers, dispersants, stabilizers, suspending agents, colorants, and flavoring agents, while for injection, buffers, preservatives, isotonic agents, solubilizers, stabilizers, etc. may be mixed and used, and for topical administration, bases, diluents, lubricants, and preservatives may be used. The formulation of the pharmaceutical composition of the present invention can be variously manufactured by mixing with a pharmaceutically acceptable carrier as described above. For example, for oral administration, it can be manufactured in the form of tablets, troches, capsules, elixirs, suspensions, syrups, wafers, etc., and for injection, it can be manufactured in the form of a single dose ampoule or multiple dose forms. Meanwhile, suitable carriers, excipients, and diluents for formulation include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, or mineral oil. Additionally, it can include fillers, anticoagulants, lubricants, wetting agents, fragrances, emulsifiers, and preservatives. The pharmaceutical composition of the present invention may be administered via the oral, intravenous, intramuscular, intra-arterial, intra-osseous, intrathecal, intracardiac, transdermal, subcutaneous, intraperitoneal, intranasal, intestinal, topical, sublingual, or rectal routes, but is preferably non-parenteral administration as described above. The non-parenteral administration refers to subcutaneous, intradermal, intravenous, intramuscular, intra-articular, intra-synovial, mediastinal, intrathecal, intra-lesional, and intracranial injection or infusion techniques. The pharmaceutical composition of the present invention may also be administered in the form of suppositories for rectal administration. The dosage of the pharmaceutical composition of the present invention can vary depending on several factors, including the activity of the specific compound used, age, body weight, general health, sex, race, administration time, route of administration, excretion rate, drug combination, and severity of the specific disease to be prevented or treated, but can be appropriately selected by a person skilled in the art. Administration may be once a day, or may be administered multiple times. The dosage should not be interpreted in any way to limit the scope of the present invention.

The artificial nucleic acid or nucleic acid stabilization composition of the present invention can be used as an alternative to various existing disease treatment methods using DNA.

Hereinafter, the present disclosure will be described in detail with reference to examples.

### [Advantageous Effects]

Since the mRNA stabilization method and mRNA stabilization composition of the present invention have enhancement effect of the target nucleic acid stabilization and target protein expression in mRNA vaccines, etc., so it is expected to be widely used in the field of health/medicine.

### [Description of Drawings]

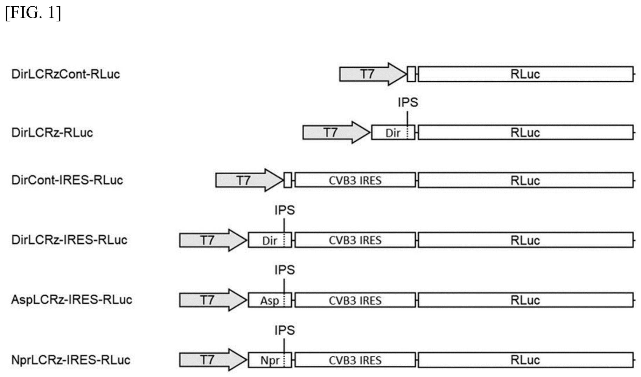

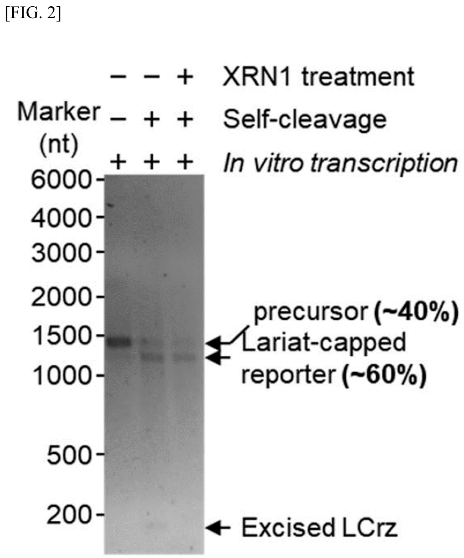

Fig. 1 shows a schematic diagram of the plasmid design of the test groups used in the present invention. Specifically, "Dir-LCrz-Cont-RLuc" refers to RNA having an analog of the cap structure present at the 5' end of the conventional mRNA, "Dir-LCrz-RLuc" refers to RNA in which the lariat-cap structure is induced at the 5' end by ribozyme having sequence represented by SEQ ID NOs: 1, "Dir-Cont-IRES-RLuc" refers to RNA having an analog of the cap structure present at the 5' end of the conventional mRNA and additionally inserted with the Coxsackievirus B3 (CVB3) IRES having sequence represented by SEQ ID NOs: 4, "Dir-LCrz-IRES-RLuc" refers to RNA in which the lariat-cap structure is induced at the 5' end by ribozyme having having sequence represented by SEQ ID NOs: 1 and additionally inserted with the Coxsackievirus B3 (CVB3) IRES having sequence represented by SEQ ID NOs: 4, "Asp-LCrz-IRES-RLuc" refers to RNA in which the lariat-cap structure is induced at the 5' end by ribozyme having sequence represented by SEQ ID NOs: 2 and additionally inserted with the Coxsackievirus B3 (CVB3) IRES having sequence represented by SEQ ID NOs: 4, and "Npr-LCrz-IRES-RLuc" refers to RNA in which the lariat-cap structure is induced at the 5' end by ribozyme having sequence represented by SEQ ID NOs: 3 and additionally inserted with the Coxsackievirus B3 (CVB3) IRES having sequence represented by SEQ ID NOs: 4. The symbols are applied identically to Figures 2 to 6 below, and the symbol "-" in the middle of the symbols may be omitted.
Fig. 2 shows the successful production of lariat-capped RNA from the plasmid of this invention.
Fig. 3a to Fig. 3c show the stability of RNA produced from the plasmid of this invention and the expression level of RLuc reporter protein.
Fig. 4 shows a comparison of the production efficiency of "Dir-LCrz-RLuc" and "Dir-LCrz-IRES-RLuc" with or without the introduction of IRES.
Fig. 5a to Fig. 5d show a comparison of the RLuc RNA formation efficiency of lariat-capped RNA using ribozyme represented by SEQ ID NOs: 1, SEQ ID NOs: 2, or SEQ ID NOs: 3.
Fig. 6a and Fig. 6b show a comparison of the intracellular RNA stability and protein translation efficiency of lariat-capped RNA using ribozyme represented by SEQ ID NOs: 1, SEQ ID NOs: 2, or SEQ ID NOs: 3.

### [Best Mode]

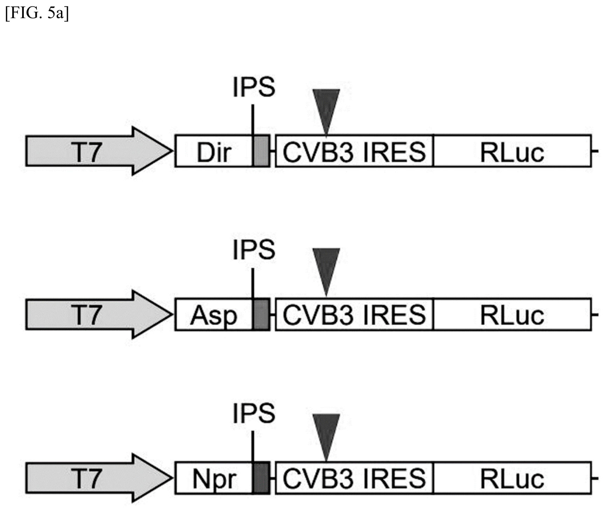

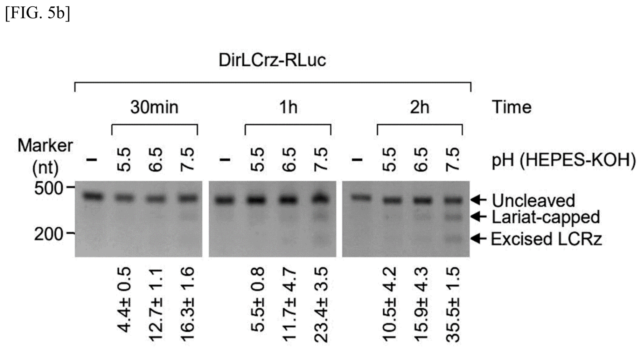

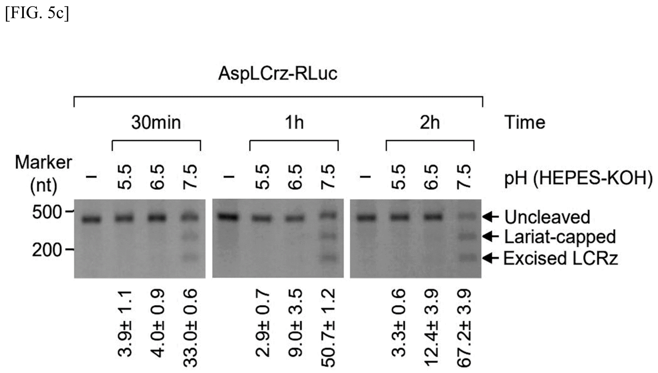

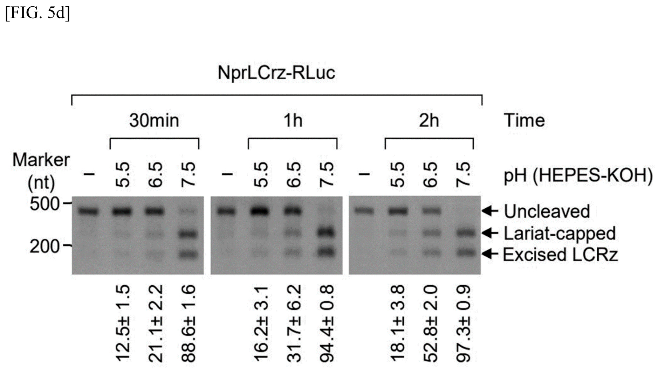

To produce a lariat-cap with better RNA stability and translation efficiency, we searched for new ribozymes, and selected two ribozymes, one from *Allovahlkampfia spelaea* (SEQ ID NO: 2) and the other from *Naegleria pringsheimi* (SEQ ID NO: 3). Using the GIR1 branching ribozyme (SEQ ID NO: 1) from Example 1 as a control, we compared the functions of the two new ribozymes. The experimental results showed that all three ribozymes have the ability to produce RNA with a lariat-cap, but the ribozyme derived from *Naegleria pringsheimi* (SEQ ID NO: 3) showed a very high efficiency of 97.3% in forming a lariat-cap under optimized pH conditions (Fig. 5a to Fig. 5d). This indicates a higher potential for use of the ribozyme from *Naegleria pringsheimi* than the GIR ribozyme used as a control.

### [Mode for Invention]

Hereinafter, the present disclosure will be described in detail with reference to the following examples. However, the following examples are merely illustrative of the present disclosure, and the content of the present disclosure is not limited by the following examples.

### Example 1. Synthesis of nucleic acid containing GIR1 branching ribozyme and confirmation of its activity

To confirm the stability of RNA with a lariat-cap compared to the cap structure (m7G) found at the 5' end of typical mRNA, a plasmid containing the GIR1 branching ribozyme (SEQ ID NOs: 1), known to form a lariat-cap structure, was synthesized. The expression gene used was RLuc (Renilla-luciferin 2-monooxygenase), and the specific plasmid structure is shown in Fig. 1. In vitro transcription was performed using T7 promoter and T7 RNA polymerase, and the synthesized RNA was analyzed using denaturing agarose gel. The results showed that full-length RNA was synthesized as expected through the in vitro transcription process. In addition, by optimizing the buffer composition in which the ribozyme is easy to work, more than 60% of the lariat-capped RNA formation efficiency was observed. That is, a novel RNA with a short length of less than 200 nucleotides (ribozyme itself nucleotide sequence) and expected to be a lariat-capped RNA was produced, which means that the RNA was split into two by the function of the GIR1 ribozyme. In particular, when treated with XRN1, a 5'-to-3' exoribonuclease, the RNA with a short length of less than 200 nucleotides disappeared significantly, while the RNA corresponding to the lariat-capped RNA was not significantly affected. Since the lariat-capped RNA does not have a 5' end, its resistance to XRN1 treatment means that the RNA produced under the experimental conditions is a lariat-capped RNA without a 5' end. The results are shown in Fig. 2.

### Example 2. Evaluation of the Nucleic Acid Stabilization Effect of Lariat-Capped RNA

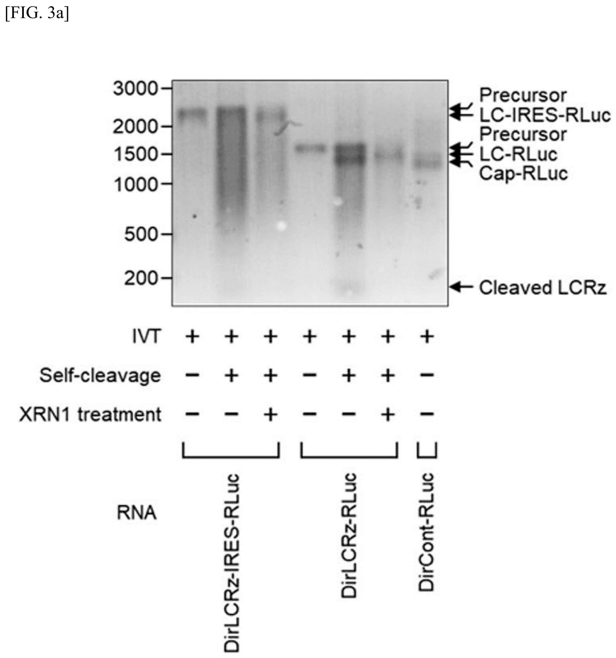

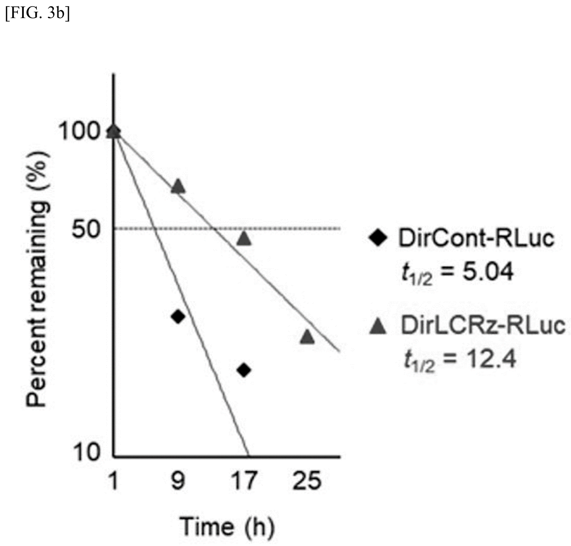

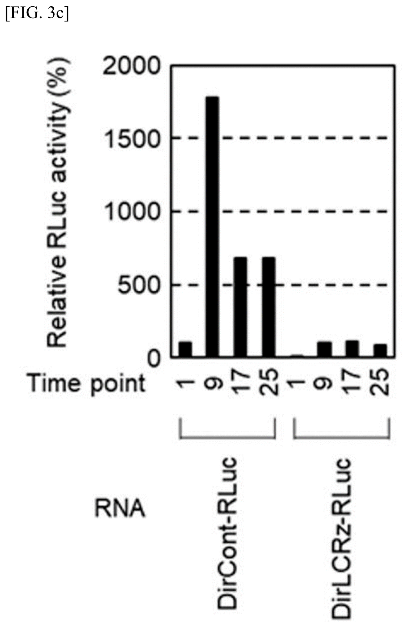

Using the plasmid structure from Example 1, RLuc (Renilla-luciferin 2-monooxygenase) genes were expressed, with one RNA having a 5' cap structure (capped RLuc RNA) and the other having a lariat-cap structure (lariat-capped RLuc RNA). The stability and protein translation efficiency of these RNAs were investigated in HeLa cells. The results showed that the capped RLuc RNA had a half-life of approximately 5 hours, while the lariat-capped RLuc RNA had a half-life of approximately 12 hours. This indicates that the lariat-capped RNA has higher stability compared to the capped RNA. However, when protein expression was evaluated using RLuc activity, the capped RLuc RNA showed high translation efficiency, while the lariat-capped RLuc RNA had almost background levels of RLuc activity due to the lack of an IRES, indicating low protein translation efficiency. The results are shown in Fig. 3a to Fig. 3c.

### Example 3. Production of nucleic acids containing reporter RNA and lariat-capped RNA and verification of their activity

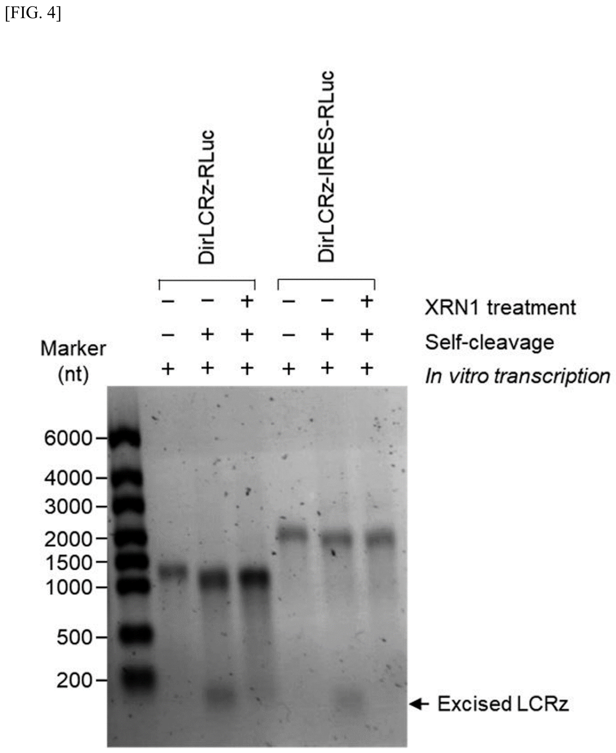

As part of the efforts to overcome the limitations of inefficient protein translation of lariat-capped RNA, a reporter RNA containing an IRES (SEQ ID NOs: 4) was constructed in front of the RLuc gene. As a result, despite the length of the RNA being sufficiently increased by introducing Coxsackievirus B3 (CVB3) IRES, it was confirmed that a large amount of RNA was produced when synthesizing RNA using the method constructed in this invention. The results are shown in Fig. 4.

### Example 4. Comparison of the effects of various types of lariat-cap formation-inducing ribozymes

In order to manufacture a more RNA stable and highly translated lariat-cap, novel ribozymes were explored and selected as Allovahlkampfia spelaea-derived ribozyme (SEQ ID NOs: 2) and Naegleria pringsheimi-derived ribozyme (SEQ ID NOs: 3). The GIR1 branching ribozyme (SEQ ID NOs: 1) in Example 1 was used as a control to compare the functions of the two novel ribozymes. Lariat-capped IRES RLUC RNA, a form of IRES insertion into lariat-capped RNA using the novel ribozymes, was prepared to compare the translation efficiency between the ribozymes. The results showed that the Naegleria pringsheimi ribozyme produced lariat-capped IRES RLUC RNA at a higher efficiency than the other two ribozymes. Therefore, it was expected to be able to sufficiently replace the conventional GIR1 ribozyme (SEQ ID NOs: 1). The results are presented in Fig. 5a to Fig. 5d.

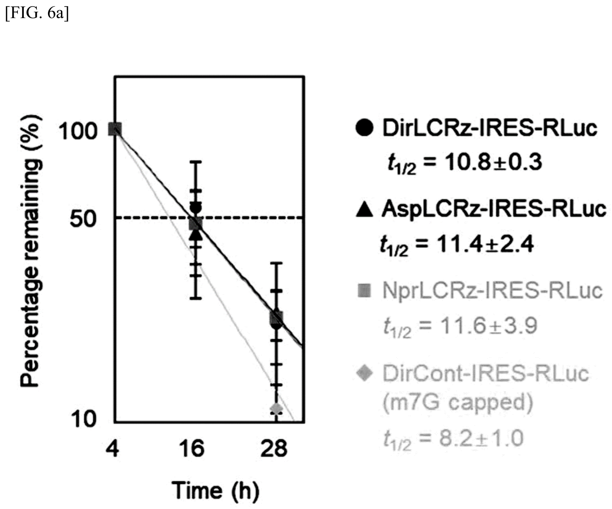

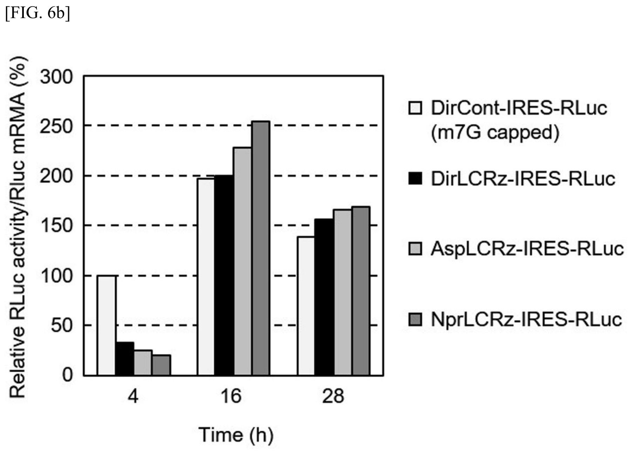

The total of 4 IRES RLuc RNAs (capped RNA, and 3 different ribozymes-synthesized Lariat-capped IRES RLuc RNA) were introduced into HeLa cells to compare the half-life of each RNA and protein synthesis efficiency within the cell. The results are presented in Fig. 6a and Fig. 6b. Experimental results showed that capped IRES RLuc RNA had a half-life of approximately 8 hours, while lariat-capped IRES RLuc RNA synthesized through 3 different ribozymes had a half-life of approximately 11 hours. In terms of protein efficiency, all the lariat-capped IRES RLuc RNA synthesized through the 3 different ribozymes used were found to have a similar translation efficiency to capped RNA. This result means that lariat-capped IRES RNA is significantly more stable in the cell than the commonly used capped RNA, while having a similar protein efficiency.

Although the present disclosure has been described in detail with reference to the specific features, it will be apparent to those skilled in the art that this description is only of a preferred embodiment thereof, and does not limit the scope of the present disclosure. Thus, the substantial scope of the present disclosure will be defined by the appended claims and equivalents thereto.

### [Industrial Applicability]

The present invention relates to a technology for remarkably improving intracellular stability and biogenesis of mRNA. An mRNA stabilization method and an mRNA stabilization composition, of the present invention, have the excellent effects of enhancing target nucleic acid stabilization and target protein expression in mRNA vaccines and various genetic diseases and metabolic diseases caused by protein deficiencies, and thus are expected to be widely used in the health/medical field.

### [Sequence Lists Free Text]

SEQ ID NOs: 1. GIR1 branching ribozyme from *Didymium iridis* ggttgggttgggaagtatcatggctaatcaccatgatgcaatcgggttgaacacttaattgggttaaa 
acggtgggggacgatcccgtaacatccgtcctaacggcgacagactgcacggccctgcctcttaggtgtgttc

aatgaacagtcgttccgaaaggaagcatccggtatcccaagacaatc
SEQ ID NOs: 2. Ribozyme from *Allovahlkampfia spelaea* 
gggcgactttctttttctcttgtgcaatggggtttatgagttaattagccaaaacgggaccttaaaaag
gtgtaagtaaccgtactaagttcgtaagaacggaatgtctagagactacacggctgagcgatttagctctcata
aatggatagtcctcagtataccatctgagcatcccatacaaaatggttaaatatttc
SEQ ID NOs: 3. Ribozyme from *Naegleria pringsheimi* 
ggtccctgttattgaggacgttttagtgtgcaatggggttcacacctttatttgccaaaacgggacctc
tgttgaggttataaaattctaacgaattgaatattccgtactaaggatttaatccggaacgtctagagactacacg
gcaaaccatattggtggtgtgaatggatagtccctagtaaccatctaggcatcccatacaaaatggtaaccata
aaa
SEQ ID NOs: 4. Internal ribosome entry site (IRES)

ttaaaacagcctgtgggttgatcccacccacaggcccattgggcgctagcactctggtatcacggta
cctttgtgcgcctgttttataccccctcccccaactgtaacttagaagtaacacacaccgatcaacagtcagcgt
ggcacaccagccacgttttgatcaagcacttctgttaccccggactgagtatcaatagactgctcacgcggttg
aaggagaaagcgttcgttatccggccaactacttcgaaaaacctagtaacaccgtggaagttgcagagtgtttc
gctcagcactaccccagtgtagatcaggtcgatgagtcaccgcattccccacgggcgaccgtggcggtggct
gcgttggcggcctgcccatggggaaacccatgggacgctctaatacagacatggtgcgaagagtctattgag
ctagttggtagtcctccggcccctgaatgcggctaatcctaactgcggagcacacaccctcaagccagagggc
agtgtgtcgtaacgggcaactctgcagcggaaccgactactttgggtgtccgtgtttcattttattcctatactgg
ctgcttatggtgacaattgagagatcgttaccatatagctattggattggccatccggtgactaatagagctatta
tatatccctttgttgggtttataccacttagcttgaaagaggttaaaacattacaattcattgttaagttgaatacag
caaaatg

## Claims

1. A method for stabilizing a desired nucleic acid, the method comprising steps of:
(a) inserting a sequence for lariat-cap formation on the 5' side of the desired nucleic acid sequence; and
(b) inserting a sequence coding for an internal ribosome entry site (IRES) between the lariat-cap formation sequence and the desired nucleic acid sequence.

2. The method according to claim 1, wherein the desired nucleic acid is an RNA sequence.

3. The method according to claim 1, wherein the sequence for lariat-cap formation is selected from the group consisting of SEQ ID NO: 1 to 3.

4. The method according to claim 1, wherein the sequence coding for IRES is a sequence known in the art or represented by SEQ ID NO: 4.

5. The method according to claim 1, wherein the stabilizing a nucleic acid is the intracellular stability of desired nucleic acids.

6. The method according to claim 1, wherein the method is for improving the stability of an RNA vaccine.

7. An artificial nucleic acid comprising:
a sequence for lariat-cap formation; and
a sequence coding for an internal ribosome entry site (IRES).

8. The artificial nucleic acid according to claim 7, wherein the sequence for lariat-cap formation is selected from the group consisting of SEQ ID NO: 1 to 3.

9. The artificial nucleic acid according to claim 7, wherein the sequence coding for IRES is a sequence known in the art or represented by SEQ ID NO: 4.

10. A composition for stabilizing a desired nucleic acid comprising the artificial nucleic acid according to claim 7.

11. The composition according to claim 10, wherein the composition is for improving the stability of mRNA vaccines or therapeutic mRNAs.

12. A composition for enhancing the immunity comprising the artificial nucleic acid according to claim 7.

13. The composition according to claim 12, wherein the composition is for a vaccine composition.

14. A vector comprising the artificial nucleic acid according to claim 7.

15. The vector according to claim 14, wherein the vector is a plasmid vector or a viral vector.

16. A transgenic organism transformed with the vector according to claim 14.

17. The transgenic organism according to claim 16, wherein the transgenic organism is a virus, bacteria, plant cell, or animal cell.

18. A pharmaceutical composition for preventing or treating a disease comprising the artificial nucleic acid according to claim 7.

19. The pharmaceutical composition according to claim 18, wherein the disease is a genetic disorder caused by genetic defect.
